(19) Europäisches Patentamt
European Patent Office
Office européen des brevets

(11) **EP 2 354 845 B1**

(12) **EUROPÄISCHE PATENTSCHRIFT**

(45) Veröffentlichungstag und Bekanntmachung des Hinweises auf die Patenterteilung:
**23.12.2015 Patentblatt 2015/52**

(21) Anmeldenummer: **10001008.1**

(22) Anmeldetag: **02.02.2010**

(51) Int Cl.:
**G03F 7/00** *(2006.01)*  **G03F 7/027** *(2006.01)*
**C07C 271/48** *(2006.01)*  **C07C 275/28** *(2006.01)*
**C07C 275/30** *(2006.01)*  **C07C 323/20** *(2006.01)*
**C07C 323/43** *(2006.01)*  **G03H 1/02** *(2006.01)*

(54) **Photopolymer-Formulierung zur Herstellung holographischer Medien**

Photopolymer Composition for the Manufacturing of Holographic Media

Composition de Photopolymère pour la Fabrication de Médias Holographiques

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(43) Veröffentlichungstag der Anmeldung:
**10.08.2011 Patentblatt 2011/32**

(73) Patentinhaber: **Covestro Deutschland AG**
**51373 Leverkusen (DE)**

(72) Erfinder:
• **Rölle, Thomas**
**51381 Leverkusen (DE)**
• **Bruder, Friedrich-Karl**
**47802 Krafeld (DE)**
• **Fäcke, Thomas**
**51375 Leverkusen (DE)**

• **Weiser, Marc-Stephan**
**51379 Leverkusen (DE)**
• **Hönel, Dennis**
**53909 Zülpich-Wichterich (DE)**

(74) Vertreter: **Levpat**
**c/o Covestro AG**
**Alfred-Nobel-Straße 10**
**40789 Monheim am Rhein (DE)**

(56) Entgegenhaltungen:
EP-A1- 1 676 870     EP-A2- 0 343 476
WO-A1-95/05408     WO-A2-2004/077511
JP-A- 2003 012 727     JP-A- 2007 101 743

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Photopolymer-Formulierung umfassend Matrixpolymere, Photoinitiatoren und (Meth) Acrylat Schreibmonomere sowie die Verwendung der Photopolymer-Formulierung zur Herstellung holographischer Medien.

Photopolymer-Formulierungen sind im Stand der Technik bekannt. So ist beispielsweise in der WO 2008/125229 A1 eine Photopolymer-Formulierung beschrieben, die Polyurethan-basierte Matrixpolymere, ein Schreibmonomer auf Acrylatbasis sowie Photoinitiatoren enthält. Im ausgehärtetem Zustand sind in der Polyurethanmatrix das Schreibmonomer und die Photoinitiatoren räumlich isotrop verteilt eingebettet.

Für die Verwendungen von Photopolymer-Formulierungen spielt die durch die holographische Belichtung im Photopolymer erzeugte Brechungsindexmodulation $\Delta n$ die entscheidende Rolle. Bei der holographischen Belichtung wird das Interferenzfeld aus Signal- und Referenzlichtstrahl (im einfachsten Fall das zweier ebener Wellen) durch die lokale Photopolymerisation von z.B. hochbrechenden Acrylate an Orten hoher Intensität im Interferenzfeld in ein Brechungsindexgitter abgebildet. Das Brechungsindexgitter im Photopolymeren (das Hologramm) enthält alle Information des Signallichtstrahls. Durch Beleuchtung des Hologramms nur mit dem Referenzlichtstrahl kann dann das Signal wieder rekonstruiert werden. Die Stärke des so rekonstruierten Signals im Verhältnis zur Stärke des eingestrahlten Referenzlichts wird Beugungseffizienz, im folgenden DE wie Diffraction Efficiency, genannt.

Im einfachsten Fall eines Hologramms, das aus der Überlagerung zweier ebener Wellen entsteht ergibt sich die DE aus dem Quotienten der Intensität des bei der Rekonstruktion abgebeugten Lichtes und der Summe der Intensitäten aus eingestrahltem Referenzlicht und abgebeugtem Licht. Je höher die DE desto effizienter ist ein Hologramm in Bezug auf die Lichtmenge des Referenzlichtes die notwendig ist, um das Signal mit einer festen Helligkeit sichtbar zu machen.

Hochbrechende Acrylate sind in der Lage, Brechungsindexgitter mit hoher Amplitude zwischen Bereichen mit niedrigem Brechungsindex und Bereichen mit hohem Brechungsindex zu erzeugen und damit in Photopolymer-Formulierungen Hologramme mit hohem DE und hohem $\Delta n$ zu ermöglichen. Dabei ist zu beachten, dass DE vom Produkt aus $\Delta n$ und der Photopolymerschichtdicke d abhängt. Je größer das Produkt desto größer die mögliche DE (für Reflexionshologramme). Die Breite des Winkelbereiches bei dem das Hologramm z.B. bei monochromatischer Beleuchtung sichtbar (rekonstruiert) wird, hängt nur von der Schichtdicke d ab.

Bei Beleuchtung des Hologramms mit z.B. weißem Licht hängt die Breite des spektralen Bereiches, der zur Rekonstruktion des Hologramms beitragen kann, ebenfalls nur von der Schichtdicke d ab. Dabei gilt je kleiner d desto größer die jeweiligen Akzeptanzbreiten. Will man daher helle und leicht sichtbare Hologramme herstellen, ist ein hohes $\Delta n$ und eine geringe Dicke d anzustreben und zwar so, dass DE möglichst groß wird. Das heißt je höher $\Delta n$ wird, desto mehr Freiraum zur Gestaltung der Schichtdicke d für helle Hologramme erreicht man ohne Verlust an DE. Daher kommt der Optimierung von $\Delta n$ bei der Optimierung von Photopolymer-Formulierungen eine herausragenden Bedeutung zu (P. Hariharan, Optical Holography, 2nd Edition, Cambridge University Press, 1996).

Aus der WO 2008/125229 A1 sind Photopolymer-Formulierungen bekannt, die hochmolekulare, mono- und difunktionelle Schreibmonomere enthalten. In Medien aus diesen Formulierungen können Hologramme geschrieben werden, die sich beispielsweise gut für die Datenspeicherung eignen. Allerdings treten bei der Herstellung und Verarbeitung der Formulierungen Probleme auf: So weisen die enthaltenen Schreibmonomere eine große Viskosität bzw. hohe $T_G$-Werte auf. Dies bedingt, dass eine gleichmäßige Verteilung der Schreibmonomere in der Photopolymer-Formulierung und einem daraus hergestellten Medium schwierig zu realisieren ist. Außerdem kann es bei Verwendung der bekannten Formulierungen in der Polymermatrix zur Bildung von Schreibmonomer-Agglomeraten kommen, was die Qualität der Medien bzw. der darin eingeschriebenen Hologramme erheblich beeinträchtigt.

JP 2007 101743 A beschreibt eine Zusammensetzung für optische Aufzeichnungen, welches ein Matrixpolymer gebildet aus einem Polyol und einem Prepolymer mit endständiger Isocyanatgruppe, und ein polymersierbares Monomer mit einer ethylenisch-ungesättigten Bindung und einer funktionellen Gruppe, welche eine Wasserstoff-Brückenbindung eingehen kann, enthält.

Aufgabe der vorliegenden Erfindung war es daher, eine Verbindung mit vergleichsweise niedriger $T_G$ und guter Löslichkeit in der Polymermatrix bereit zu stellen, die leicht als Schreibmonomer in einer Photopolymer-Formulierung homogen verteilt werden kann und mit der gleichzeitig helle Hologramme erhalten werden können.

Diese Aufgabe ist durch den Anspruch 1 gelöst.

Hologramme, die beispielsweise in Filme aus der erfindungsgemäßen Photopolymer-Formulierung einbelichtet werden, zeichnen sich durch ihre hohe Helligkeit aus.

Die Matrixpolymere können insbesondere Polyurethane sein.

Vorzugsweise sind die Polyurethane durch Umsetzung einer Isocyanatkomponente a) mit einer Isocyanatreaktiven Komponente b) erhältlich.

Die Isocyanatkomponente a) umfasst bevorzugt Polyisocyanate. Als Polyisocyanate können alle dem Fachmann an sich gut bekannten Verbindungen oder deren Mischungen eingesetzt werden, die im Mittel

zwei oder mehr NCO-Funktionen pro Molekül aufweisen. Diese können auf aromatischer, araliphatischer, aliphatischer oder cycloaliphatischer Basis sein. In untergeordneten Mengen können auch Monoisocyanate und/oder ungesättigte Gruppen enthaltende Polyisocyanate mitverwendet werden.

Beispielsweise geeignet sind Butylendiisocyanat, Hexamethylendiisocyanat (HDI), Isophorondiisocyanat (IPDI), 1,8-Diisocyanato-4-(isocyanatomethyl)-octan, 2,2,4- und/oder 2,4,4-Trimethylhexamethylendiisocyanat, die isomeren Bis-(4,4'-isocyanatocyclohexyl)methan und deren Mischungen beliebigen Isomerengehalts, Isocyanatomethyl-1,8-octandiisocyanat, 1,4-Cyclohexylendiisocyanat, die isomeren Cyclohexandimethylendiisocyanate, 1,4-Phenylendiisocyanat, 2,4- und/oder 2,6-Toluylendiisocyanat, 1,5-Naphthylendiisocyanat, 2,4'- oder 4,4'-Diphenylmethandiisocyanat und/oder Triphenylmethan-4,4',4"-triisocyanat.

Ebenfalls möglich ist der Einsatz von Derivaten monomerer Di- oder Triisocyanate mit Urethan-, Harnstoff-, Carbodiimid-, Acylharnstoff-, Isocyanurat-, Allophanat-, Biuret-, Oxadiazintrion-, Uretdion- und/oder Iminooxadiazindionstrukturen.

Bevorzugt ist der Einsatz von Polyisocyanaten auf Basis aliphatischer und/oder cycloaliphatischer Di- oder Triisocyanate.

Besonders bevorzugt handelt es sich bei den Polyisocyanaten der Komponente a) um di- oder oligomerisierte aliphatische und/oder cycloaliphatische Di- oder Triisocyanate.

Ganz besonders bevorzugt sind Isocyanurate, Uretdione und/oder Iminooxadiazindione basierend auf HDI, 1,8-Diisocyanato-4-(isocyanatomethyl)-octan oder deren Mischungen.

Ebenfalls können als Komponente a) NCO-funktionelle Prepolymere mit Urethan-, Allophanat-, Biuret- und/oder Amidgruppen eingesetzt werden. Prepolymere der Komponente a) werden in dem Fachmann an sich gut bekannter Art und Weise durch Umsetzung von monomeren, oligomeren oder Polyisocyanaten a1) mit isocyanatreaktiven Verbindungen a2) in geeigneter Stöchiometrie unter optionalem Einsatz von Katalysatoren und Lösemitteln erhalten.

Als Polyisocyanate a1) eignen sich alle dem Fachmann an sich bekannten aliphatischen, cycloaliphatischen, aromatischen oder araliphatischen Di- und Triisocyanate, wobei es unerheblich ist, ob diese mittels Phosgenierung oder nach phosgenfreien Verfahren erhalten wurden. Daneben können auch die diem Fachmann an sich gut bekannten höhermolekularen Folgeprodukte monomerer Di- und/oder Triisocyanate mit Urethan-, Harnstoff, Carbodiimid-, Acylharnstoff-, Isocyanurat-, Allophanat-, Biuret-, Oxadiazintrion-, Uretdion-, Iminooxadiazindionstruktur jeweils einzeln oder in beliebigen Mischungen untereinander eingesetzt werden.

Beispiele für geeignete monomere Di- oder Triisocyanate, die als Komponente a1) eingesetzt werden können, sind Butylendiisocyanat, Hexamethylendiisocyanat (HDI), Isophorondiisocyanat (IPDI), Trimethyl-hexamethylen-diisocyanat (TMDI), 1,8-Diisocyanato-4-(isocyanatomethyl)-octan, Isocyanatomethyl-1,8-octandiisocyanat (TIN), 2,4- und/oder 2,6-Toluen-diisocyanat.

Als isocyanatreaktive Verbindungen a2) zum Aufbau der Prepolymere werden bevorzugt OH- funktionelle Verbindungen eingesetzt. Diese sind analog den OH-funktionellen Verbindungen wie sie nachfolgend für die Komponente b) beschrieben sind.

Ebenfalls möglich ist der Einsatz von Aminen zur Prepolymerherstellung. Beispielsweise geeignet sind Ethylendiamin, Diethylentriamin, Triethylentetramin, Propylendiamin, Diaminocyclohexan, Diaminobenzol, Diaminobisphenyl, difunktionelle Polyamine wie z.B. die Jeffamine®, aminterminierte Polymere mit zahlenmittleren Molmassen bis 10000 g/Mol oder deren beliebige Gemische untereinander.

Zur Herstellung von biuretgruppenhaltigen Prepolymeren wird Isocyanat im Überschuss mit Amin umgesetzt, wobei eine Biuretgruppe entsteht. Als Amine eignen sich in diesem Falle für die Umsetzung mit den erwähnten Di-, Tri- und Polyisocyanaten alle oligomeren oder polymeren, primären oder sekundären, difunktionellen Amine der vorstehend genannten Art.

Bevorzugte Prepolymere sind Urethane, Allophanate oder Biurete aus aliphatischen Isocyanat-funktionellen Verbindungen und oligomeren oder polymeren Isocyanat-reaktiven Verbindungen mit zahlenmittleren Molmassen von 200 bis 10000 g/Mol, besonders bevorzugt sind Urethane, Allophanate oder Biurete aus aliphatischen Isocyanat-funktionellen Verbindungen und oligomeren oder polymeren Polyolen oder Polyaminen mit zahlenmittleren Molmassen von 500 bis 8500 g/Mol und ganz besonders bevorzugt sind Allophanate aus HDI oder TMDI und difunktionellen Polyetherpolyolen mit zahlenmittleren Molmassen von 1000 bis 8200 g/Mol.

Bevorzugt weisen die vorstehend beschriebenen Prepolymere Restgehalte an freiem monomeren Isocyanat von weniger als 1 Gew.-%, besonders bevorzugt weniger als 0.5 Gew.-% und ganz besonders bevorzugt weniger als 0.2 Gew.-% auf.

Selbstverständlich kann die Isocyanatkomponente anteilsmäßig neben den beschriebenen Prepolymeren weitere Isocyanatkomponenten enthalten. Hierfür kommen aromatische, araliphatische, aliphatische und cycloaliphatische Di-, Tri- oder Polyisocyanate in Betracht. Es können auch Mischungen solcher Di-, Tri- oder Polyisocyanate eingesetzt werden. Beispiele geeigneter Di-, Tri- oder Polyisocyanate sind Butylendiisocyanat, Hexamethylendiisocyanat (HDI), Isophorondiisocyanat (IPDI), 1,8-Diisocyanato-4-(isocyanatomethyl)octan, 2,2,4- und/oder 2,4,4-Trimethylhexamethylendiisocyanat (TMDI), die isomeren Bis(4,4'-isocyanatocyclohexyl)methane und deren Mischungen beliebigen Isomerengehalts, Isocyanatomethyl-1,8-octandiisocyanat, 1,4-Cyclohexylendiisocyanat, die isomeren Cyclohexandimethylendiisocyanate, 1,4-Phenylendiisocyanat, 2,4- und/oder 2,6-Toluylendiisocyanat, 1,5-Naphthylendiisocyanat, 2,4'- oder 4,4'-Diphenylmethandiisocyanat, Triphenylmethan-4,4',4"-triisocyanat oder deren Derivate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur und Mischungen derselben. Bevorzugt sind Polyisocyanate auf Basis oligomerisierter und/oder derivatisierter Diisocyanate, die durch geeignete Verfahren von überschüssigem Diisocyanat befreit wurden, insbesondere die des Hexamethylendiisocyanat. Besonders bevorzugt sind die oligomeren Isocyanurate, Uretdione und Iminooxadiazindione des HDI sowie deren Mischungen.

Es ist gegebenenfalls auch möglich, dass die Isocyanatkomponente a) anteilsmäßig Isocyanate enthält, die teilweise mit isocyanat-reaktiven ethylenisch ungesättigten Verbindungen umgesetzt sind. Bevorzugt werden hierbei als isocyanat-reaktives ethylenisch ungesättigte Verbindungen $\alpha,\beta$-ungesättigte Carbonsäurederivate wie Acrylate, Methacrylate, Maleinate, Fumarate, Maleimide, Acrylamide, sowie Vinylether, Propenylether, Allylether und Dicyclopentadienyl-Einheiten enthaltende Verbindungen, die mindestens eine gegenüber Isocyanaten reaktive Gruppe aufweisen, eingesetzt. Besonders bevorzugt sind dies Acrylate und Methacrylate mit mindestens einer isocyanatreaktiven Gruppe. Als hydroxyfunktionelle Acrylate oder Methacrylate kommen beispielsweise Verbindungen wie 2-Hydroxyethyl(meth)acrylat, Polyethylenoxid-mono(meth)acrylate, Polypropylenoxidmono(meth)acrylate, Polyalkylenoxidmono(meth)acrylate, Poly($\varepsilon$-caprolacton)mono(meth)acrylate, wie z.B. Tone® M100 (Dow, USA), 2-Hydroxypropyl(meth)acrylat, 4-Hydroxybutyl(meth)acrylat, 3-Hydroxy-2,2-dimethylpropyl(meth)acrylat, die hydroxyfunktionellen Mono-, Di- oder Tetra(meth)acrylate mehrwertiger Alkohole wie Trimethylolpropan, Glycerin, Pentaerythrit, Dipentaerythrit, ethoxyliertes, propoxyliertes oder alkoxyliertes Trimethylolpropan, Glycerin, Pentaerythrit, Dipentaerythrit oder deren technische Gemische in Betracht. Darüberhinaus sind isocyanat-reaktive oligomere oder polymere ungesättigte Acrylat- und/oder Methacrylatgruppen enthaltende Verbindungen alleine oder in Kombination mit den vorgenannnten monomeren Verbindungen geeignet. Der Anteil an Isocyanaten an der Isocyanatkomponente a), die teilweise mit isocyanat-reaktiven ethylenisch ungesättigten Verbindungen umgesetzt sind beträgt 0 bis 99 %, bevorzugt 0 bis 50 %, besonders bevorzugt 0 bis 25 % und ganz besonders bevorzugt 0 bis 15 %.

Es ist gegebenenfalls auch möglich, dass die vorgenannten Isocyanatkomponente a) vollständig oder anteilsmäßig Isocyanate enthält, die ganz oder teilweise mit dem Fachmann aus der Beschichtungstechnologie bekannten Blockierungsmitteln umgesetzt sind. Als Beispiel für Blockierungsmittel seien genannt: Alkohole, Lactame, Oxime, Malonester, Alkylacetoacetate, Triazole, Phenole, Imidazole, Pyrazole sowie Amine, wie z.B. Butanonoxim, Diisopropylamin, 1,2,4-Triazol, Dimethyl-1,2,4-triazol, Imidazol, Malonsäurediethylester, Acetessigester, Acetonoxim, 3,5-Dimethylpyrazol, $\varepsilon$-Caprolactam, N-tert.-Butyl-benzylamin, Cyclopentanoncarboxyethylester oder beliebige Gemische dieser Blockierungsmittel.

Als Komponente b) können an sich alle polyfunktionellen, isocyanatreaktiven Verbindungen eingesetzt werden, die im Mittel wenigstens 1.5 isocyanatreaktive Gruppen pro Molekül aufweisen.

Isocyanatreaktive Gruppen im Rahmen der vorliegenden Erfindung sind bevorzugt Hydroxy-, Amino- oder Thiogruppen, besonders bevorzugt sind Hydroxyverbindungen.

Geeignete polyfunktionelle, isocyanatreaktive Verbindungen sind beispielsweise Polyester-, Polyether-, Polycarbonat-, Poly(meth)acrylat- und/oder Polyurethanpolyole.

Als Polyesterpolyole sind beispielsweise lineare Polyesterdiole oder verzweigte Polyesterpolyole geeignet, wie sie in bekannter Weise aus aliphatischen, cycloaliphatischen oder aromatischen Di- bzw. Polycarbonsäuren bzw. ihren Anhydriden mit mehrwertigen Alkoholen einer OH-Funktionalität $\geq$ 2 erhalten werden.

Beispiele für solche Di- bzw. Polycarbonsäuren bzw. Anhydride sind Bernstein-, Glutar-, Adipin-, Pimelin-, Kork-, Azelain-, Sebacin-, Nonandicarbon-, Decandicarbon-, Terephthal-, Isophthal-, o-Phthal-, Tetrahydrophthal-, Hexahydrophthal- oder Trimellitsäure sowie Säureanhydride wie o-Phthal-, Trimellit- oder Bernsteinsäureanhydrid oder deren beliebige Gemische untereinander.

Beispiele für geeignete Alkohole sind Ethandiol, Di-, Tri-, Tetraethylenglykol, 1,2-Propandiol, Di-, Tri-, Tetrapropylenglykol, 1,3-Propandiol, Butandiol-1,4, Butandiol-1,3, Butandiol-2,3, Pentandiol-1,5, Hexandiol-1,6, 2,2-Dimethyl-1,3-propandiol, 1,4-Dihydroxycyclohexan, 1,4-Dimethylolcyclohexan, Octandiol-1,8, Decandiol-1,10, Dodecandiol-1,12, Trimethylolpropan, Glycerin oder deren beliebige Gemische untereinander.

Die Polyesterpolyole können auch auf natürlichen Rohstoffen wie Rizinusöl basieren. Ebenfalls möglich ist, dass die Polyesterpolyole auf Homo- oder Mischpolymerisaten von Lactonen basieren, wie sie bevorzugt durch Anlagerung von Lactonen bzw. Lactongemischen wie Butyrolacton, $\varepsilon$-Caprolacton und/oder Methyl-$\varepsilon$-caprolacton an hydroxyfunktionelle Verbindungen wie mehrwertige Alkohole einer OH-Funktionalität $\geq$ 2 beispielsweise der vorstehend genannten Art erhalten werden können.

Solche Polyesterpolyole haben bevorzugt zahlenmittlere Molmassen von 400 bis 4000 g/Mol, besonders bevorzugt von 500 bis 2000 g/Mol. Ihre OH-Funktionalität beträgt bevorzugt 1.5 bis 3.5, besonders bevorzugt 1.8 bis 3.0.

Geeignete Polycarbonatpolyole sind in an sich bekannter Weise durch Umsetzung von organischen Carbonaten oder Phosgen mit Diolen oder Diol-Mischungen zugänglich.

Geeignete organische Carbonate sind Dimethyl-, Diethyl- und Diphenylcarbonat.

Geeignete Diole bzw. Mischungen umfassen die an sich im Rahmen der Polyestersegmente genannten mehrwertigen Alkoholen einer OH-Funktionalität $\geq$ 2, bevorzugt 1,4-Butandiol, 1,6-Hexandiol und/oder 3-Methylpentandiol, oder auch Polyesterpolyole können zu Polycarbonatpolyolen umgearbeitet werden.

Solche Polycarbonatpolyole haben bevorzugt zahlenmittlere Molmassen von 400 bis 4000 g/Mol, besonders bevorzugt von 500 bis 2000 g/Mol. Die OH-Funktionalität dieser Polyole beträgt bevorzugt 1.8 bis 3.2, besonders bevorzugt 1.9 bis 3.0.

Geeignete Polyetherpolyole sind gegebenenfalls blockweise aufgebaute Polyadditionsprodukte cyclischer Ether an OH- oder NH-funktionelle Startermoleküle.

Geeignete cyclische Ether sind beispielsweise Styroloxide, Ethylenoxid, Propylenoxid, Tetrahydrofuran, Butylenoxid, Epichlorhydrin, sowie ihre beliebigen Mischungen.

Als Starter können die an sich im Rahmen der Polyesterpolyole genannten mehrwertigen Alkohole einer OH-Funktionalität $\geq$ 2 sowie primäre oder sekundäre Amine und Aminoalkohole verwendet werden.

Bevorzugte Polyetherpolyole sind solche der vorgenannten Art ausschließlich basierend auf Propylenoxid oder statistische oder Block-Copolymere basierend auf Propylenoxid mit weiteren 1-Alkylenoxide, wobei der 1-Allykenoxidanteil nicht höher als 80 Gew.-% ist. Daneben sind Poly(trimethylenoxid)e gemäß Formel (III) sowie Mischungen der als bevorzugt genannten Polyole bevorzugt. Besonders bevorzugt sind Propylenoxid-Homopolymere sowie statistische oder Block-Copolymere, die Oxyethylen-, Oxypropylen- und/oder Oxybutyleneinheiten aufweisen, wobei der Anteil der Oxypropyleneinheiten bezogen auf die Gesamtmenge aller Oxyethylen-, Oxypropylen- und Oxybutyleneinheiten mindestens 20 Gew.-%, bevorzugt mindestens 45 Gew.-% ausmacht. Oxypropylen- und Oxybutylen umfasst hierbei alle jeweiligen linearen und verzweigten C3- und C4-Isomere.

Solche Polyetherpolyole haben bevorzugt zahlenmittlere Molmassen von 250 bis 10000 g/Mol, besonders bevorzugt von 500 bis 8500 g/Mol und ganz besonders bevorzugt von 600 bis 4500 g/Mol. Die OH-Funktionalität beträgt bevorzugt 1.5 bis 4.0, besonders bevorzugt 1.8 bis 3.1.

Daneben sind als Bestandteile der Komponente b) als polyfunktionelle, isocyanatreaktive Verbindungen auch niedermolekulare, d.h. mit Molekulargewichten kleiner 500 g/mol, kurzkettige, d.h. 2 bis 20 Kohlenstoffatome enthaltende aliphatische, araliphatische oder cycloaliphatische di, tri oder polyfunktionelle Alkohole geeignet.

Dies können beispielsweise sein Ethylenglykol, Diethylenglykol, Triethylenglykol, Tetraethylenglykol, Dipropylenglykol, Tripropylenglykol, 1,2-Propandiol, 1,3-Propandiol, 1,4-Butandiol, Neopentylglykol, 2-Ethyl-2-butylpropandiol, Trimethylpentandiol, stellungsisomere Diethyloctandiole, 1,3-Butylenglykol, Cyclohexandiol, 1,4-Cyclohexandimethanol, 1,6-Hexandiol, 1,2- und 1,4-Cyclohexandiol, hydriertes Bisphenol A (2,2-Bis(4-hydroxycyclohexyl)propan), 2,2-Dimethyl-3-hydroxypropionsäure (2,2-dimethyl-3-hydroxypropylester). Beispiele geeigneter Triole sind Trimethylolethan, Trimethylolpropan oder Glycerin. Geeignete höherfunktionelle Alkohole sind Ditrimethylolpropan, Pentaerythrit, Dipentaerythrit oder Sorbit.

Die eingesetzten Photoinitiatoren sind üblicherweise durch aktinische Strahlung aktivierbare Initiatoren, die eine Polymerisation der entsprechenden polymerisierbaren Gruppen auslösen. Photoinitiatoren sind an sich bekannte, kommerziell vertriebene Verbindungen, wobei zwischen unimolekularen (Typ I) und bimolekularen (Typ II) Initiatoren unterschieden wird. Desweiteren werden diese Initiatoren je nach chemischer Natur für die radikalische, die anionische (oder), die kationische (oder gemischte) Formen der vorgenannten Polymerisationen eingesetzt.

Die Photoinitiatoren können insbesondere einen anionischen, kationischen oder neutralen Farbstoff und einen Coinitiator umfassen.

(Typ I)-Systeme für die radikalische Photopolymerisation sind z.B. aromatische Ketonverbindungen, z.B. Benzophenone in Kombination mit tertiären Aminen, Alkylbenzophenone, 4,4'-Bis(dimethylamino)benzophenon (Michlers Keton), Anthron und halogenierte Benzophenone oder Mischungen der genannten Typen. Weiter geeignet sind (Typ II)-Initiatoren wie Benzoin und seine Derivate, Benzilketale, Acylphosphinoxide z.B. 2,4,6-Trimethyl-benzoyl-diphenylphosphinoxid, Bisacylophosphinoxide, Phenylglyoxylsäureester, Campherchinon, alpha-Aminoalkylphenon, alpha-,alpha-Dialkoxyacetophenon, 1-[4-(Phenylthio)phenyl]octan-1,2-dion-2-(O-benzoyloxim) und alpha-Hydroxyalkylphenol.

Auch die in EP-A 0223587 beschriebenen Photoinitiatorsysteme bestehend aus einer Mischung aus einem Ammoniumarylborat und einem oder mehreren Farbstoffen können als Photoinitiator eingesetzt werden. Als Ammoniumarylborat eignen sich beispielsweise Tetrabutylammonium Triphenylhexylborat, Tetrabutylammonium Triphenylbutylborat, Tetrabutylammonium Trinapthylhexylborat, Tetrabutylammonium Tris(4-tert.butyl)-phenylbutylborat, Tetrabutylammonium Tris-(3-fluorphenyl)-hexylborat, Tetramethylammonium Triphenylbenzylborat, Tetra(n-hexyl)ammonium (sec-Butyl)triphenylborat, 1-Methyl-3-octylimidazolium Dipentyldiphenylborat und Tetrabutylammonium Tris-(3-Chlor-4-methylphenyl)-hexylborat. Als Farbstoffe eignen sich beispielsweise Neu-Methylenblau, Thionin, Basic Yellow, Pinacynol Chlorid, Rhodamin 6G, Gallocyanin, Ethylviolett, Victoria Blue R, Celestine Blue, Chinaldinrot, Kristallviolett, Brilliant Grün, Astrazon Orange G, Darrow Red, Pyronin Y, Basic Red 29, Pyrillium I, Cyanin und Methylenblau, Azur A (Cunningham et al., RadTech'98 North America UV/EB Conference Proceedings, Chicago, Apr. 19-22, 1998).

Die für die anionische Polymerisation verwendeten Photoinitiatoren sind in der Regel (Typ I)-Systeme und leiten sich von Übergangsmetall-Komplexen der ersten Reihe ab. Hier sind Chrom-Salze, wie z.B. trans-Cr(NH$_3$)$_2$(NCS)$_4^-$ (Kutal et al, Macromolecules 1991, 24, 6872) oder Ferrocenyl-Verbindungen (Yamaguchi et al. Macromolecules 2000, 33, 1152). Eine weitere Möglichkeit der anionischen Polymerisation besteht in der Verwendung von Farbstoffen, wie Kristallviolett Leukonitril oder Malchit Grün Leukonitril, die durch photolytischen Zerfall Cyanoacrylate polymerisieren können (Neckers et al. Macromolecules 2000, 33, 7761). Allerdings wird dabei das Chromophor in das Polymer eingebaut, so dass die resultierenden Polymere durchgefärbt sind.

Die für die kationische Polymerisation verwendeten Photoinitiatoren bestehen im wesentlichen aus drei Klassen: Aryldiazonium-Salze, Onium-Salze (hier speziell: Iodonium-, Sulfonium- und Selenonium-Salze) sowie Organometall-Verbindungen. Phenyldiazonium-Salze können unter Bestrahlung sowohl in Gegenwart als auch in Abwesenheit eines Wasserstoff-Donors ein Kation erzeugten, dass die Polymerisation initiiert. Die Effizienz des Gesamtsystems wird durch die Natur des verwendeten Gegenions zur Diazonium-Verbindung bestimmt. Bevorzugt sind hier die wenig reaktiven aber recht teuren SbF$_6^-$, AsF$_6^-$ oder PF$_6^-$. Für den Einsatz in Beschichtung dünner Filme sind diese Verbindungen i.d.R wenig geeignet, da die den nach der Belichtung freigesetzten Stickstoff die Oberflächegüte herabgesetzt wird (pinholes) (Li et al., Polymeric Materials Science and Engineering, 2001, 84, 139).

Sehr weit verbreitet und auch in vielerlei Form kommerziell erhältlich sind Onium-Salze, speziell Sulfonium- und Iodonium-Salze. Die Photochemie dieser Verbindungen ist nachhaltig untersucht worden. Die Iodonium-Salze zerfallen nach der Anregung zunächst homolytisch und erzeugen somit ein Radikal und ein Radikalanion, welches sich durch H-Abstraktion stabilisiert und ein Proton freisetzt und dann die kationische Polymerisation startet (Dektar et al. J. Org. Chem. 1990, 55, 639; J. Org. Chem., 1991, 56. 1838). Dieser Mechanimus ermöglicht den Einsatz von Iodonium-Salzen ebenfalls für die radikalische Photopolymerisation. Hierbei kommt erneut der Wahl des Gegenions eine große Bedeutung zu, bevorzugt werden ebenfalls SbF$_6^-$, AsF$_6^-$ oder PF$_6^-$. Ansonsten ist in dieser Strukturklasse die Wahl der Substitution des Aromaten recht frei und im wesentlichen durch die Verfügbarkeit geeigneter Startbausteine für die Synthese bestimmt.

Bei den Sulfonium-Salzen handelt es sich um Verbindungen, die in nach Norrish(II) zerfallen (Crivello et al., Macromolecules, 2000, 33, 825). Auch bei den Sulfonium-Salzen kommt der Wahl des Gegenions eine kritische Bedeutung zu, die sich im Wesentlichen in der Härtungsgeschwindigkeit der Polymere äußert. Die besten Ergebnisse werden i.d.R. mit SbF$_6^-$ Salzen erzielt.

Da die Eigenabsorption von Iodonium- und Sulfonium-Salze bei <300nm liegt, müssen diese Verbindungen für die Photopolymerisation mit nahem UV oder kurzwelligem sichtbarem Licht entsprechend sensibilisiert werden. Dies gelingt durch die Verwendung von höher absorbierenden Aromaten wie z.B. Anthracen und Derivaten (Gu et al., Am. Chem. Soc. Polymer Preprints, 2000,41 (2), 1266) oder Phenothiazin bzw. dessen Derivate (Hua et al, Macromolecules 2001, 34, 2488-2494).

Es kann vorteilhaft sein, Gemische dieser Verbindungen einzusetzen. Je nach zur Härtung verwendeter Strahlungsquelle muss Typ und Konzentration an Photoinitiator in dem Fachmann bekannter Weise angepasst werden. Näheres ist zum Beispiel in P. K. T. Oldring (Ed.), Chemistry & Technology of UV & EB Formulations For Coatings, Inks & Paints, Vol. 3, 1991, SITA Technology, London, S. 61 - 328 beschrieben.

Bevorzugte Photoinitiatoren sind Mischungen aus Tetrabutylammonium Tetrahexylborat, Tetrabutylammonium Triphenylhexylborat, Tetrabutylammonium Tris-(3-fluorphenyl)-hexylborat ([191726-69-9], CGI 7460, Produkt der Ciba Inc, Basel) und Tetrabutylammonium Tris-(3-Chlor-4-methylphenyl)-hexylborat ([1147315-11-4], CGI 909, Produkt der Ciba Inc, Basel) mit Farbstoffen wie beispielsweise Astrazon Orange G, Methylenblau, Neu Methylenblau, Azur A, Pyrillium I, Safranin O, Cyanin, Gallocyanin, Brilliant Grün, Kristallviolett, Ethylviolett und Thionin.

Es ist vorgesehen, dass die Photopolymer-Formulierung zusätzlich Urethane als Weichmacher enthält, wobei die Urethane mit wenigstens einem Fluoratom substituiert sind.

Bei einer weiteren bevorzugten Ausführungsform ist vorgesehen, dass die Schreibmonomere zusätzlich ein weiters mono- oder multifunktionales Schreibmonomer umfassen, wobei es sich insbesondere um ein mono- oder multifunktionelles Acrylat handeln kann.

Ein weiterer Gegenstand der Erfindung ist die Verwendung einer erfindungsgemäßen Photopolymer-Formulierung zur Herstellung holographischer Medien, die durch entsprechende Belichtungsprozesse für optische Anwendungen im gesamten sichtbaren und nahen UV-Bereich (300-800 nm) zu Hologrammen verarbeitet werden können. Visuelle Hologramme umfassen alle Hologramme, die nach dem Fachmann bekannten Verfahren aufgezeichnet werden können. Darunter fallen unter anderem In-Line (Gabor) Hologramme, Off-Axis Hologramme, Full-Aperture Transfer Hologramme, Weißlicht-Transmissionshologramme ("Regenbogenhologramme), Denisyukhologramme, Off-Axis Reflektionshologramme, Edge-Lit Hologramme sowie holographische Stereogramme. Bevorzugt sind Reflexionshologramme, Denisyukhologramme, Transmissionshologramme.

Mögliche optische Funktionen der Hologramme, die mit den erfindungsgemäßen Photopolymer-Formulierungen hergestellt werden können entsprechen den optische Funktionen von Lichtelementen wie Linsen, Spiegel, Umlenkspiegel, Filter, Streuscheiben, Beugungselemente, Lichtleiter, Lichtlenker (waveguides), Projektionsscheiben und/oder Masken. Häufig zeigen diese optischen Elemente eine Frequenzselektivität, je nachdem wie die Hologramme belichtet wurden und welche Dimensionen das Hologramm hat.

Zudem können mittels der erfindungsgemäßen Photopolymer-Formulierungen auch holographische Bilder oder Darstellungen hergestellt werden, wie zum Beispiel für persönliche Portraits, biometrische Darstellungen in Sicherheitsdokumenten, oder allgemein von Bilder oder Bildstrukturen für Werbung, Sicherheitslabels, Markenschutz, Markenbranding, Etiketten, Designelementen, Dekorationen, Illustrationen, Sammelkasten, Bilder und dergleichen sowie Bilder, die digitale Daten repräsentieren können u.a. auch in Kombination mit den zuvor dargestellen Produkten. Holographische Bilder können den Eindruck eines dreidimensionalen Bildes haben, sie können aber auch Bildsequenzen, kurze Filme oder eine Anzahl von verschiedenen Objekten darstellen, je nachdem aus welchem Winkel, mit welcher (auch bewegten) Lichtquelle etc. diese beleuchtet wird. Aufgrund dieser vielfältigen Designmöglichkeiten stellen Hologramme, insbesondere Volumenhologramme, eine attraktive technische Lösung für die oben genannten Anwendung dar.

Die Photopolymere-Formulierungen können insbesondere als holografisches Medium in Form eines Films verwendet werden. Dabei wird als Träger eine Lage eines für Licht im sichtbaren Spektralbereich (Transmission größer als 85% im Wellenlängenbereich von 400 bis 780 nm) transparenten Materials oder Materialverbunds ein- oder beidseitig beschichtet sowie ggf. eine Abdeckschicht auf der oder den Photopolymerlagen appliziert.

Bevorzugte Materialien oder Materialverbünde des Trägers basieren auf Polycarbonat (PC), Polyethylenterephthalat (PET), Polybutylenterephthalat, Polyethylen, Polypropylen, Celluloseacetat, Cellulosehydrat, Cellulosenitrat, Cycloolefinpolymere, Polystyrol, Polyepoxide, Polysulfon, Cellulosetriacetat (CTA), Polyamid, Polymethylmethacrylat, Polyvinylchlorid, Polyvinylbutyral oder Polydicyclopentadien oder deren Mischungen. Besonders bevorzugt basieren sie auf PC, PET und CTA. Materialverbünde können Folienlaminate oder Coextrudate sein. Bevorzugte Materialverbünde sind Duplex- und Triplex-folien aufgebaut nach einem der Schemata A/B, A/B/A oder A/B/C. Besonders bevorzugt sind PC/PET, PET/PC/PET und PC/TPU (TPU = Thermoplastisches Polyurethan).

Alternativ zu den vorgenannten Kunststoffträgern können auch planare Glasplatten eingesetzt werden, die insbesondere für großflächige abbildungsgenaue Belichtungen Verwendung finden, z.B. für holographische Lithographie (Ng, Willie W.; Hong, Chi-Shain; Yariv, Amnon. Holographic interference lithography for integrated optics. IEEE Transactions on Electron Devices (1978), ED-25(10), 1193-1200, ISSN:0018-9383).

Die Materialien oder Materialverbünde des Trägers können einseitig oder beidseitig antihaftend, antistatisch, hydrophobiert oder hydrophiliert ausgerüstet sein. Die genannten Modifikationen dienen an der Photopolymerschicht zugewandten Seite dem Zweck, dass die Photopolymerlage von dem Träger zerstörungsfrei abgelöst werden kann. Eine Modifikation der der Photopolymerlage abgewandten Seite des Trägers dient dazu, dass die erfindungsgemäßen Medien speziellen mechanischen Anforderungen genügen, die z.B. bei der Verarbeitung in Rollenlaminatoren, insbesondere bei Rolle-zu-Rolle-Verfahren, gefordert sind.

## Beispiele

Die Erfindung wird im Folgenden anhand von Beispielen näher erläutert.

Sofern nicht abweichend vermerkt beziehen sich alle Prozentangaben auf Gewichtsprozent.

## Messmethoden:

Die Messung des Brechungsindex erfolgte bei einer Wellenlänge von 405 nm. Der Brechungsindex n in Abhängigkeit von der Wellenlänge der Proben wurden aus den Transmissions- und Reflexionsspektren erhalten. Dazu wurden ca. 100 - 300 nm dicke Filme der Proben auf Quarzglasträger aus verdünnter Lösung in Butylacetat aufgeschleudert. Das Transmissions- und Reflexionsspektrum dieses Schichtpaketes wurde mit einem Spektrometer der Firma STEAG ETA-Optik, CD-Measurement System ETA-RT gemessen und danach die Schichtdicke und der spektrale Verlauf von n an die gemessenen Transmissions- und Reflexionsspektren angepasst. Dies geschieht mit der internen Software des Spektrometers und erfordert zusätzlich die n Daten des Quarzglassubstrates, die in einer Blindmessung vorab bestimmt wurden.

Zur Bestimmung der Glastemperatur $T_G$ wurden ~ 10 mg der Substanz in Aluminium Tiegel eingewogen, der mit einem gelochten Deckel verschlossen wird. Die Glastemperatur wird dann an den so präparierten Proben mit einem Differential Scanning Kalorimeter DSC822e/400 der Fa. Mettler-Toledo bestimmt. Es werden drei Aufheizzyklen mit einer Heizrate von 20 K/min gefahren. Die Start- und Endtemperatur des ersten Zyklus beträgt -100°C bzw. 80°C. Die Start und Endtemperaturen des zweiten und dritten Zykluses betragen -100°C bzw. 150°C. Die entsprechenden Kühlraten betragen 50 K/min. Der Ofen und die Probe des Kalorimeters werden mit einem Stickstoffstrom von 20 ml/min Fluss durchspült. Als $T_G$ der Probe wird die Glastemperatur beim 3. Aufheizzyklus ermittelt.

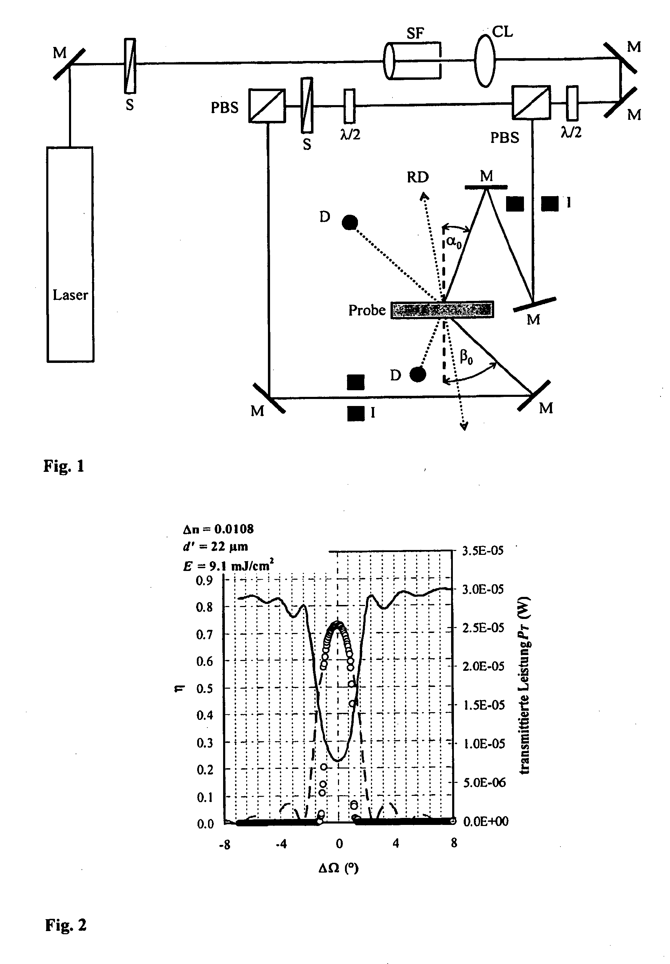

Messung der holograpischen Eigenschaften: Der Strahl eines He-Ne Lasers (Emissionswellenlänge 633 nm) wurde mit Hilfe des Raumfilter (SF) und zusammen mit der Kollimationslinse (CL) in einen parallelen homogenen Strahl umgewandelt. Die finalen Querschnitte des Signal und Referenzstrahls werden durch die Irisblenden (I) festgelegt. Der Durchmesser der Irisblendenöffnung beträgt 0.4 cm. Die polarisationsabhängigen Strahlteiler (PBS) teilen den Laserstrahl in zwei kohärente gleich polarisierte Strahlen. Über die λ/2 Plättchen wurden die Leistung des Referenzstrahls auf 0.5 mW und die Leistung des Signalstrahls auf 0.65 mW eingestellt. Die Leistungen wurden mit den Halbleiterdetektoren (D) bei ausgebauter Probe bestimmt. Der Einfallswinkel ($\alpha_0$) des Referenzstrahls beträgt -21.8°, der Einfallswinkel ($\beta_0$) des Signalstrahls beträgt 41.8°. Die Winkel werden ausgehend von der Probennormale zur Strahlrichtung gemessen. Gemäß Figur 1 hat daher $\alpha_0$ ein negatives Vorzeichen und $\beta_0$ ein positives Vorzeichen. Am Ort der Probe (Medium) erzeugte das Interferenzfeld der zwei überlappenden Strahlen ein Gitter heller und dunkler Streifen die senkrecht zur Winkelhalbierenden der zwei auf die Probe einfallenden Strahlen liegen (Reflexionshologramm). Der Streifenabstand A, auch Gitterperiode genannt, im Medium beträgt ~ 225 nm (der Brechungsindex des Mediums zu ~1.504 angenommen).

Figur 1 zeigt die Geometrie eines Holographic Media Testers (HMT) bei λ = 633 nm (He-Ne Laser): M = Spiegel, S = Verschluss, SF = Raumfilter, CL = Kollimatorlinse, λ/2 = λ/2 Platte, PBS = polarisationsempfindlicher Strahlteiler, D = Detektor, I = Irisblende, $\alpha_0$ = -21.8°, $\beta_0$ = 41.8° sind die Einfallswinkel der kohärenten Strahlen außerhalb der Probe (des Mediums) gemessen. RD = Referenzrichtung des Drehtisches.

Mit einem holographischen Versuchsaufbau wie in Figur 1 dargestellt wurden die Beugungseffizienz (DE) der Medien gemessen.

Es wurden auf folgende Weise Hologramme in das Medium geschrieben:

- Beide Shutter (S) sind für die Belichtungszeit *t* geöffnet.

- Danach wurde bei geschlossenen Shuttern (S) dem Medium 5 Minuten Zeit für die Diffusion der noch nicht polymerisierten Schreibmonomere gelassen.

Die geschriebenen Hologramme wurden nun auf folgende Weise ausgelesen. Der Shutter des Signalstrahls blieb geschlossen. Der Shutter des Referenzstrahls war geöffnet. Die Irisblende des Referenzstrahls wurde auf einen Durchmesser < 1 mm geschlossen. Damit erreichte man, dass für alle Drehwinkel ($\Omega$) des Mediums der Strahl immer vollständig im zuvor geschriebenen Hologramm lag. Der Drehtisch überstrich nun computergesteuert den Winkelbereich von $\Omega_{min}$ bis $\Omega_{max}$ mit einer Winkelschrittweite von 0.05°. $\Omega$ wird von der Probennormale zur Referenzrichtung des Drehtisches gemessen. Die Referenzrichtung des Drehtisches ergibt sich dann wenn beim Schreiben des Hologramms der Einfallswinkel des Referenz- und des Signalstrahls betragsmäßig gleich sind also $\alpha_0$ = -31.8° und $\beta_0$ = 31.8° gilt. Dann beträgt $\Omega_{recording}$ = 0°. Für $\alpha_0$ = -21.8° und $\beta_0$ = 41.8° beträgt $\Omega_{recording}$ daher 10°. Allgemein gilt für das Interferenzfeld beim Schreiben ("recording") des Hologramms:

$$\alpha_0 = \theta_0 + \Omega_{recording}.$$

$\theta_0$ ist der Halbwinkel im Laborsystem außerhalb des Mediums und es gilt beim Schreiben des Hologramms:

$$\theta_0 = \frac{\alpha_0 - \beta_0}{2}.$$

In diesem Fall gilt also $\theta_0$ = -31.8°. An jedem angefahrenen Drehwinkel D wurden die Leistungen des in der nullten Ordnung transmittierten Strahls mittels des entsprechenden Detektors D und die Leistungen des in die erste Ordnung abgebeugten Strahls mittels des Detektors D gemessen. Die Beugungseffizienz ergab sich bei jedem angefahrenen Winkel $\Omega$ als der Quotient aus:

$$\eta = \frac{P_D}{P_D + P_T}$$

$P_D$ ist die Leistung im Detektor des abgebeugten Strahls und $P_T$ ist die Leistung im Detektor des transmittierten Strahls.

Mittels des oben beschriebenen Verfahrens wurde die Braggkurve, sie beschreibt den Beugungswirkungsgrad $\eta$ in Abhängigkeit des Drehwinkels $\Omega$, des geschriebenen Hologramms gemessen und in einem Computer gespeichert. Zusätzlich wurde auch die in die nullte Ordnung transmittierte Intensität gegen den Drehwinkel $\Omega$ aufgezeichnet und in einem Computer gespeichert.

Die maximale Beugungseffizienz (DE = $\eta_{max}$) des Hologramms, also sein Spitzenwert, wurde bei $\Omega_{reconstruction}$

ermittelt. Eventuell musste dazu die Position des Detektors des abgebeugten Strahls verändert werden, um diesen maximalen Wert zu bestimmen.

Der Brechungsindexkontrast $\Delta n$ und die Dicke d der Photopolymerschicht wurde nun mittels der Coupled Wave Theorie (siehe; H. Kogelnik, The Bell System Technical Journal, Volume 48, November 1969, Number 9 Seite 2909 - Seite 2947) an die gemessene Braggkurve und den Winkelverlauf der transmittierten Intensität ermittelt. Dabei ist zu beachten, dass wegen der durch die Photopolymerisation auftretenden Dickenschwindung der Streifenabstand $\Lambda'$ des Hologramms und die Orientierung der Streifen (slant) vom Streifenabstand A des Interferenzmusters und dessen Orientierung abweichen kann. Demnach wird auch der Winkel $\alpha_0'$ bzw. der entsprechende Winkel des Drehtisches $\Omega_{reconstruction}$, bei dem maximale Beugungseffizienz erreicht wird von $\alpha_0$ bzw. vom entsprechenden $\Omega_{recording}$ abweichen. Dadurch verändert sich die Bragg-Bedingung. Diese Veränderung wird im Auswerteverfahren berücksichtigt. Das Auswerteverfahren wird im Folgenden beschrieben:

Alle geometrischen Größen, die sich auf das geschriebene Hologramm beziehen und nicht auf das Interferenzmuster werden als gestrichene Größen dargestellt.

Für die Braggkurve $\eta(\Omega)$ eines Reflexionshologramms gilt nach Kogelnik:

$$\eta = \begin{cases} \dfrac{1}{1 - \dfrac{1 - (\xi/\nu)^2}{\sin^2\left(\sqrt{\xi^2 - \nu^2}\right)}}, & \text{für } \nu^2 - \xi^2 < 0 \\[4mm] \dfrac{1}{1 + \dfrac{1 - (\xi/\nu)^2}{\sinh^2\left(\sqrt{\nu^2 - \xi^2}\right)}}, & \text{für } \nu^2 - \xi^2 \geq 0 \end{cases}$$

mit:

$$\nu = \frac{\pi \cdot \Delta n \cdot d'}{\lambda \cdot \sqrt{|c_s \cdot c_r|}}$$

$$\xi = -\frac{d'}{2 \cdot c_s} \cdot DP$$

$$c_s = \cos(\vartheta') - \cos(\psi') \cdot \frac{\lambda}{n \cdot \Lambda'}$$

$$c_r = \cos(\vartheta')$$

$$DP = \frac{\pi}{\Lambda'} \cdot \left( 2 \cdot \cos(\psi' - \vartheta') - \frac{\lambda}{n \cdot \Lambda'} \right)$$

$$\psi' = \frac{\beta' + \alpha'}{2}$$

$$\Lambda' = \frac{\lambda}{2 \cdot n \cdot \cos(\psi' - \alpha')}$$

Beim Auslesen des Hologramms ("reconstruction") gilt wie analog oben dargestellt:

$$\vartheta'_0 = \theta_0 + \Omega$$

$$\sin(\vartheta'_0) = n \cdot \sin(\vartheta')$$

An der Bragg-Bedingung ist das "Dephasing" $DP$ = 0. Und es folgt entsprechend:

$$\alpha'_0 = \theta_0 + \Omega_{reconstruction}$$

$$\sin(\alpha'_0) = n \cdot \sin(\alpha')$$

Der noch unbekannt Winkel $\beta'$ kann aus dem Vergleich der Bragg-Bedingung des Interferenzfeldes beim Schreiben des Hologramms und der Bragg-Bedingung beim Auslesen des Hologramms ermittelt werden unter der Annahme, dass nur Dickenschwindung stattfindet. Dann folgt:

$$\sin(\beta') = \frac{1}{n} \cdot \left[ \sin(\alpha_0) + \sin(\beta_0) - \sin(\theta_0 + \Omega_{reconstruction}) \right]$$

$\nu$ ist die Gitterstärke, $\xi$ ist der Detuning Parameter und $\psi'$ die Orientierung (Slant) des Brechungsindexgitters das geschrieben wurde. $\alpha'$ und $\beta'$ entsprechen den Winkeln $\alpha_0$ und $\beta_0$ des Interferenzfeldes beim Schreiben des Hologramms, aber im Medium gemessen und für das Gitter des Hologramms gültig (nach Dickenschwindung). n ist der mittlere Brechungsindex des Photopolymers und wurde zu 1.504 gesetzt. $\lambda$ ist die Wellenlänge des Laserlichts im Vakuum.

Die maximale Beugungseffizienz (DE = $\eta_{max}$) ergibt sich dann für $\xi$ = 0 zu:

$$DE = \tanh^2(\nu) = \tanh^2 \left( \frac{\pi \cdot \Delta n \cdot d'}{\lambda \cdot \sqrt{\cos(\alpha') \cdot \cos(\alpha' - 2\psi)}} \right)$$

Figur 2 zeigt die gemessene transmittierte Leistung $P_T$ (rechte $y$-Achse) als durchgezogene Linie gegen das Winkeldetuning $\Delta\Omega$ aufgetragen, die gemessene Beugungseffizienz $\eta$ (linke $y$-Achse) als ausgefüllte Kreise gegen das Winkeldetuning $\Delta\Omega$ aufgetragen (soweit die endliche Größe des Detektors es erlaubte) und die Anpassung der Kogelnik Theorie als gestrichelte Linie (linke $y$-Achse).

Die Messdaten der Beugungseffizienz, die theoretische Braggkurve und die transmittierte Intensität werden wie in Figur 2 gezeigt gegen den zentrierten Drehwinkel $\Delta\Omega \equiv \Omega_{reconstruction}-\Omega=\alpha'_0-\vartheta'_0$, auch Winkeldetuning genannt, aufgetragen.

Da DE bekannt ist wird die Form der theoretischen Braggkurve nach Kogelnik nur noch durch die Dicke $d'$ der Photopolymerschicht bestimmt. $\Delta n$ wird über DE für gegebene Dicke $d'$ so nachkorrigiert, dass Messung und Theorie von DE immer übereinstimmen. $d'$ wird nun solange angepasst bis die Winkelpositionen der ersten Nebenminima der theoretischen Braggkurve mit den Winkelpositionen der ersten Nebenmaxima der transmittierten Intensität übereinstimmen und zudem die volle Breite bei halber Höhe (FWHM) für die theoretische Braggkurve und für die transmittierte Intensität übereinstimmen.

Da die Richtung in der ein Reflexionshologramm bei der Rekonstruktion mittels eines $\Omega$-Scans mitrotiert, der Detektor für das abgebeugte Licht aber nur einen endlichen Winkelbereich erfassen kann, wird die Braggkurve von breiten Holgrammen (kleines $d'$) bei einem $\Omega$-Scan nicht vollständig erfasst, sondern nur der zentrale Bereich, bei geeigneter Detektorpositionierung. Daher wird die zur Braggkurve komplementäre Form der transmittierten Intensität zur Anpassung der Schichtdicke d' zusätzlich herangezogen.

Figur 2 zeigt die Darstellung der Braggkurve $\eta$ nach der Coupled Wave Theorie (gestrichelte Linie), des gemessenen Beugungswirkungsgrades (ausgefüllte Kreise) und der transmittierten Leistung (schwarz durchgezogene Linie) gegen das Winkeldetuning $\Delta\Omega$.

Für eine Formulierung wurde diese Prozedur eventuell mehrfach für verschiedene Belichtungszeiten $t$ an verschiedenen Medien wiederholt, um festzustellen bei welcher mittleren Energiedosis des einfallenden Laserstrahls beim Schreiben des Hologramms DE in den Sättigungswert übergeht. Die mittlere Energiedosis E ergibt sich wie folgt aus den Leistungen der zwei den Winkeln $\alpha_0$ und $\beta_0$ zugeordneten Teilstrahlen (Referenzstrahl mit $P_r = 0.50$ mW und Signalstrahl mit $P_s = 0.63$ mW), der Belichtungszeit t und dem Durchmesser der Irisblende (0.4 cm):

$$E\,(\mathrm{mJ/cm}^2) = \frac{2 \cdot \left[P_r + P_s\right] \cdot t\,(\mathrm{s})}{\pi \cdot 0.4^2\ \mathrm{cm}^2}$$

Die Leistungen der Teilstrahlen wurden so angepasst, dass in dem Medium bei den verwendeten Winkeln $\alpha_0$ und $\beta_0$, die gleiche Leistungsdichte erreicht wird.

Die angegebenen NCO-Werte (Isocyanat-Gehalte) wurden gemäß DIN EN ISO 11909 bestimmt.

Die Festgehalte wurden bestimmt, indem unter Berücksichtigung der Bedienungsanleitung der Waage ca. 1 g Substanz auf eine spezielle Einweg-Aluschale aufgetragen und bei 140°C solange erhitzt, bis 30 Sekunden Gewichtskonstanz besteht oder Systeme mit einem maximalen Lösemittelgehalt von 10 Gew.-% werden bei 160°C für 20 Minuten erhitzt.

**Substanzen:**

Die verwendeten Alkohole und Cyanurchlorid sowie Lösungsmittel und Reagenzien wurden im Chemikalienhandel bezogen.

| | |
|---|---|
| CGI-909 | Tetrabutylammonium-tris(3-chlor-4-methylphenyl)(hexyl)borat, [1147315-11-4] ist ein von der CIBA Inc., Basel, Schweiz, hergestelltes Produkt. |
| Desmorapid Z | Dibutylzinn-dilaurat [77-58-7], Produkt der Bayer MaterialScience AG, Leverkusen, Deutschland. |
| Desmodur® N 3900 | Produkt der Bayer MaterialScience AG, Leverkusen, DE, Hexandiisocyanatbasiertes Polyisocyanat, Anteil an Iminooxadiazindion mindestens 30 %, NCO-Gehalt: 23.5 %. |
| Baytec® WE 180 | Produkt der Bayer MaterialScience LLC, Pittsburgh, US, Dicyclohexandiisocyanat-basiertes Polyisocyanat, basierend auf Polytetramethylen-etherglycol, NCO-Gehalt: 18.3-18.9 %. |
| Fomrez UL 28 | Urethanisierungskatalysator, Handelsprodukt der Momentive Performance Chemicals, Wilton, CT, USA. |
| KarenzAOI® | (2-Isocyanatoethylacrylat, [13641-96-8] im Jahr 2010 erhältliches Produkt der SHOWA DENKO K.K., Fine Chemicals Group, Specialty Chemicals Department, Chemicals Division). |
| KarenzMOI® | (2-Isocyanatoethyl-methacrylat, [30674-80-7] im Jahr 2010 erhältliches Produkt der SHOWA DENKO K.K., Fine Chemicals Group, Specialty Chemicals Department, Chemicals Division). |

**Beispiel 1: 2-{[(Phenoxy)carbonyl]amino}ethylacrylat**

In einem 250 mL Rundkolben wurden 64.0 g Phenol, 0.03 g Desmorapid Z und 0.06 g 2,6-Di-tert.-butyl-4-methylphenol in 60 mL Ethylacetat vorgelegt und auf 60 °C erwärmt. Anschließend wurden 36.0 g KarenzAOI® zugetropft und die Mischung weiter auf 60 °C gehalten, bis der Isocyanatgehalt unter 0.1 % gesunken war. Danach wurde abgekühlt. Anschließend wurde das Produkt im Rotationsverdampfer vom Ethylacetat befreit. Das Produkt wurde als farbloser Feststoff erhalten.

Die in Tabelle 1 angegebenen Beispiele wurden analog aus den angegebenen Phenolen und KarenzAOI® hergestellt.

Tabelle 1: Acrylate aus KarenzAOI® und Phenolen

| Beispiel | Name | $R^4$ | $R^5$ | $R^6$ | $R^7$ | $R^8$ | $T_G$ [°C] | Beschreibung |
|---|---|---|---|---|---|---|---|---|
| 1 | 2-{[(Phenoxy)carbonyl]-amino}ethylacrylat | H | H | H | H | H | - | Farbloser Feststoff |
| 2 | 2-{[(2-Bromphenoxy)-carbonyl]amino}ethyl-acrylat | Br | H | H | H | H | - | Farbloser Feststoff |
| 3 | 2-{[(3-Bromphenoxy)-carbonyl]amino}ethylacrylat | H | Br | H | H | H | -45 | Farbloser Feststoff |
| 4 | 2-{[(4-Bromphenoxy)-carbonyl]amino}ethyl-acrylat | H | H | Br | H | H | - | Farbloser Feststoff |
| 5 | 2- {[(3,4-Dichlorphenoxy)-carbonyl]amino}ethyl-acrylat | H | Cl | Cl | H | H | -36 | Farbloser Feststoff |
| 6 | 2-{[(2-Iodphenoxy)-carbonyl]amino}ethylacrylat | I | H | H | H | H | - | Farbloser Feststoff |
| 7 | 2- {[(3-Iodphenoxy)-carbonyl]amino}ethyl-acrylat | H | I | H | H | H | - | Farbloses Öl |
| 8 | 2- {[(4-Iodphenoxy)-carbonyl]amino}ethylacrylat | H | H | I | H | H | - | Farbloser Feststoff |
| 9 | 2-{[(4-Brom-2-chlorphenoxy)carbonyl]amino} -ethylacrylat | Cl | H | Br | H | H | -32 | Farbloser Feststoff |
| 10 | 2-{[(2-Brom-4-chlorphenoxy)carbonyl]amino} -ethylacrylat | Br | H | Cl | H | H | - | Farbloser Feststoff |
| 11 | 2- {[(2,4-Dibromphenoxy)-carbonyl]amino}ethyl-acrylat | Br | H | Br | H | H | -37 | Farbloses Öl |
| 12 | 2-({[3-(Methylsulfanyl)-phenoxy]carbonyl} amino)-ethylacrylat | H | SMe | H | H | H | -44 | Farbloser Feststoff |
| 13 | 2-({[2-(Methylsulfanyl)-phenoxy]carbonyl} amino)-ethylacrylat | SMe | H | H | H | H | - | Farbloser Feststoff |
| 14 | 2-({[4-(Methylsulfanyl)-phenoxy]carbonyl} amino)-ethylacrylat | H | H | SMe | H | H | - | Farbloser Feststoff |
| 15 | 2-[(Biphenyl-4-yloxy)carbonyl]-amino}ethylacrylat | H | H | Ph | H | H | - | Farbloser kristalliner Feststoff |
| 16 | 2- {[(Biphenyl-2-yloxy)carbonyl]-amino}ethylacrylat | Ph | H | H | H | H | - | Klare viskose Flüssigkeit |
| 17 | 2-{[(Biphenyl-3-yloxy)carbonyl]-amino}ethylacrylat | H | Ph | H | H | H | -21 | Gelbbräunlicher viskose Flüssigkeit, die wachsartig kristallisiert |

**Beispiel 18: 2-[(Phenylcarbamoyl)amino]ethylacrylat**

In einem 50 mL Rundkolben wurden 9.92 g Anilin, 0.012 g Desmorapid Z und 0.025 g 2,6-Di-tert.-butyl-4-methylphenol vorgelegt und auf 60 °C erwärmt. Anschließend wurden 15.0 g KarenzAOI® zugetropft und die Mischung weiter auf 60 °C gehalten, bis der Isocyanatgehalt unter 0.1 % gesunken war. Danach wurde abgekühlt. Das Produkt wurde als farbloser Feststoff erhalten.

Die in Tabelle 2 angebenen Beispiele wurden analog aus den angegebenen Anilinen und KarenzAOI® hergestellt.

Tabelle 2: Acrylate aus KarenzAOI® und Anilinen

| Beispiel | Name | X | $R^4$ | $R^5$ | $R^6$ | $R^7$ | $R^8$ | $T_G$ [°C] | Beschreibung |
|---|---|---|---|---|---|---|---|---|---|
| 18 | 2-[(Phenylcarbamoyl)-amino]ethylacrylat | N-H | H | H | H | H | H | - | Farbloser Feststoff |
| 19 | 2-{[Methyl(phenyl)-carbamoyl]amino}-ethylacrylat | N-Me | H | H | H | H | H | -41 | Farbloser Feststoff |
| 20 | 2- {[(3-Bromphenyl)-(methyl)carbamoyl]-amino}ethylacrylat | N-Me | H | Br | H | H | H | -33 | Farblose Flüssigkeit |
| 21 | 2-{[(3-Chlorphenyl)-(methyl)carbamoyl]-amino} ethylacrylat | N-Me | H | Cl | H | H | H | -35 | Farblose Flüssigkeit |
| 22 | 2-[(3-Chlorphenyl)-(ethyl)carbamoyl]-amino}ethylacrylat | N-Et | H | Cl | H | H | H | - | Farbloser Feststoff |
| 23 | 2- {[(2-Fluorphenyl)-(methyl)carbamoyl]-amino}ethylacrylat | N-Me | F | H | H | H | H | -39 | Farblose Flüssigkeit |
| 24 | 2-{[(4-Bromphenyl)-(methyl)carbamoyl]-amino}ethylacrylat | N-Me | H | H | Br | H | H | -29 | Farblose Flüssigkeit |
| 25 | 2- {[(4-Chlorphenyl)-(ethyl)carbamoyl]-amino}ethylacrylat | N-Et | H | H | Cl | H | H | -37 | Farblose Flüssigkeit |
| 26 | 2- {[(4-Chlorphenyl)-(methyl)carbamoyl]-amino}ethylacrylat | N-Me | H | H | Cl | H | H | - | Farblose Flüssigkeit |
| 27 | 2-({Methyl[3-(methyl-sulfanyl)phenyl]-carbamoyl}amino)-ethylacrylat | N-Me | H | SMe | H | H | H | - | Farbloses Öl |

**Beispiel 28: 2-{[(Phenoxy)carbonyl]amino}ethylmethacrylat**

In einem 250 mL Rundkolben wurden 14.1 g Phenol, 19 mg Dibutylzinndilaurat und 40 mg 2,6-Di-tert.-butyl-4-methylphenol in 37.5 mL Ethylacetat vorgelegt und auf 60 °C erwärmt und langsam Luft durchgeleitet. Anschließend wurden 23.3 g 2-Isocyanatoethylmethacrylat (KarenzMOI®) langsam zugetropft und die Mischung weiter auf 60 °C gehalten, bis der Isocyanatgehalt im IR nicht mehr nachweisbar war. Danach wurde abgekühlt und das Lösungsmittel am Rotationsverdampfer entfernt. Das Produkt wurde als farbloser Feststoff erhalten.

Die in Tabelle 3 angegebenen Beispiele wurden analog aus den angegebenen Phenolen und KarenzMOI® hergestellt.

Tabelle 3: Methacrylate aus KarenzMOI® und Phenolen

| Beispiel | Name | $R^4$ | $R^5$ | $R^6$ | $R^7$ | $R^8$ | $T_G$ [°C] | Beschreibung |
|---|---|---|---|---|---|---|---|---|
| 28 | 2-{[(Phenoxy)-carbonyl]amino}ethylmethacrylat | H | H | H | H | H | - | Weißer, kristalliner Feststoff |
| 29 | 2-{[(3-Bromphenoxy)-carbonyl]amino}ethyl-methacrylat | H | Br | H | H | H | -41 | Gelblich, kristalliner Feststoff |
| 30 | 2-{[(3,4-Dichloro-phenoxy)carbonyl]-amino}ethylacrylat | H | Cl | Cl | H | H | - | Weißer, kristalliner Feststoff |
| 31 | 2-{[(3-Iodophenoxy)-carbonyl]amino}ethylmethacrylat | H | I | H | H | H | -27 | Weißer, kristalliner Feststoff |
| 32 | 2- {[(3-Chloro-4-bromo-phenoxy)carbonyl]-amino}ethylmethacrylat | H | Cl | Br | H | H | - | Gelblich, kristalliner Feststoff |
| 33 | 2- {[(2-Bromo-4-chlorophenoxy)carbonyl]-amino} ethylmethacrylat | Br | H | Cl | H | H | -20 | Weißer, kristalliner Feststoff |
| 34 | 2- {[(2,4-Dibromophenoxy)carbonyl]-amino} ethylacrylat | Br | I | Br | H | H | -38 | Farbloser amorpher Feststoff, langsam kristallisierend |
| 35 | 2-[(3-Methylthiophenoxy)carbonyl]-amino}ethylmethacrylat | H | SMe | H | H | H | -39 | Rosa, kristalliner Feststoff |
| 36 | 2- {[(4-Phenylphenoxy)-carbonyl]amino}ethyl-methacrylat | H | H | Ph | H | H | - | hellgelber, kristalliner Feststoff |
| 37 | 2-{[(2-Phenylphenoxy)-carbonyl]amino} ethylmethacrylat | Ph | H | H | H | H | - | Klare viskose Flüssigkeit |
| 38 | 2-{[(3-Phenylphenoxy)-carbonyl]amino}ethyl-methacrylat | H | Ph | H | H | H |  | Cremefarbener kristalliner Feststoff |

**Beispiel 39: 2-{[Methyl(phenyl)carbamoyl]amino}ethyl2-methylprop-2-enoat**

In einem Rundkolben wurden 9.6 g N-Methylanilin, 0.012 g Desmorapid Z und 0.024 g 2,6-Di-tert.-butyl-4-methylphenol in 23.6 g Ethylacetat vorgelegt und auf 60 °C erwärmt. Anschließend wurden 14.0 g KarenzMOI® zugetropft und die Mischung weiter auf 60 °C gehalten, bis der Isocyanatgehalt unter 0.1 % gesunken war. Danach wurde abgekühlt und im Rotationsverdampfer von dem Ethylacetat befreit. Das Produkt wurde als gelbes Flüssigkeit erhalten.

Die in Tabelle 4 angegebenen Beispiele wurden analog aus den angegebenen Anilinen und KarenzMOI® hergestellt.

Tabelle 4: Methacrylate aus KarenzAOI® und Anilinen

| Beispiel | Name | X | R⁴ | R⁵ | R⁶ | R⁷ | R⁸ | $T_G$ [°C] | Beschreibung |
|---|---|---|---|---|---|---|---|---|---|
| 39 | 2- {[Methyl (Phenyl)-carbamoyl]amino}2-methylprop-2-enoat | N-Me | H | H | H | H | H | -37 | Gelbe Flüssigkeit |
| 40 | 2-{[(3-Chlorophenyl)-(methyl)carbamoyl]-amino}ethyl 2-methylprop-2-enoat | N-Me | H | Cl | H | H | H | -24 | Dunkelbraune Flüssigkeit |
| 41 | 2- [(2-Fluorophenyl)-(methyl)carbamoyl]-amino}ethyl2-methylprop-2-enoat | N-Me | F | H | H | H | H | -31 | Violette, klare Flüssigkeit |
| 42 | 2- {[(4-Bromophenyl)-(methyl)carbamoyl]-amino} ethyl 2-methylprop-2-enoat | N-Me | H | H | Br | H | H | - | Weißer, kristalliner Feststoff |
| 43 | 2- {[(4-Chlorophenyl)-(ethyl)carbamoyl]-amino}ethyl 2-methyl-prop-2-enoat | N-Et | H | H | Cl | H | H | -31 | Gelblich, kristalliner Feststoff |
| 44 | 2- {[(4-Chlorophenyl)-(methyl)carbamoyl]-amino}ethyl 2-methylprop-2-enoat | N-Me | H | H | Cl | H | H | - | Gelblich, kristalliner Feststoff |

**Herstellung des Vergleichsbeispiels 1: Phosphorothioyltris(oxybenzol-4,1-diylcarbamoyloxyethan-2,1-diyl)trisacrylat [1072455-04-9]**

In einem 500 mL Rundkolben wurden 0.1 g 2,6-Di-tert.-butyl-4-methylphenol, 0.05 g Dibutylzinndilaurat (Desmorapid Z, Bayer MaterialScience AG, Leverkusen, Deutschland) sowie und 213.07 g einer 27 %-igen Lösung von Tris(p-isocyanatophenyl)thiophosphat in Ethylacetat (Desmodur® RFE, Produkt der Bayer MaterialScience AG, Leverkusen, Deutschland) vorgelegt und auf 60 °C erwärmt. Anschließend wurden 42.37 g 2-Hydroxyethylacrylat zugetropft und die Mischung weiter auf 60 °C gehalten, bis der Isocyanatgehalt unter 0.1 % gesunken war. Danach wurde abgekühlt und im Vakuum das Ethylacetat vollständig entfernt. Das Produkt wurde als teilkristalliner Feststoff erhalten. Das erhaltene Produkt hat eine $T_G$ von + 19 °C

Zur Prüfung der optischen Eigenschaften wurden wie im Folgenden beschrieben Medien hergestellt und optisch vermessen:

**Herstellung der Polyol-Komponente 1:**

In einem 1 L Kolben wurden 0.18 g Zinnoctoat, 374.8 g ε-Caprolacton und 374.8 g eines difunktionellen Polytetrahydrofuranpolyetherpolyols (Equivalentgewicht 500 g/Mol OH) vorgelegt und auf 120 °C aufgeheizt und so lange auf dieser Temperatur gehalten, bis der Festgehalt (Anteil der nicht-flüchtigen Bestandteile) bei 99.5 Gew.-% oder darüber lag. Anschließend wurde abgekühlt und das Produkt als wachsiger Feststoff erhalten.

**Herstellung der Polyol-Komponente 2:**

In einem 1 L Kolben wurden 0.18 g Zinnoctoat, 374.8 g ε-Caprolacton und 374.8 g eines difunktionellen Polytetrahydrofuranpolyetherpolyols (Equivalentgewicht 650 g/Mol OH) vorgelegt und auf 120 °C aufgeheizt und so lange auf dieser Temperatur gehalten, bis der Festgehalt (Anteil der nicht-flüchtigen Bestandteile) bei 99.5 Gew.-% oder darüber lag. Anschließend wurde abgekühlt und das Produkt als wachsiger Feststoff erhalten.

**Medium 1:**

5.927 g der wie oben beschrieben hergestellten Polyol-Komponente wurden mit 2.50 g Acrylat aus Beispiel 5, 0.10 g CGI 909, 0.010 g Neu Methylenblau und 0.35 g N-Ethylpyrilidon bei 60 °C gemischt, so dass eine klare Lösung erhalten wurde. Anschließend wurde auf 30 °C abgekühlt, 1.098 g Desmodur® N 3900 zugegeben und erneut gemischt. Schließlich wurden 0.006 g Fomrez UL 28 zugegeben und erneut kurz gemischt. Die erhaltene, flüssige Masse wurde dann auf eine Glasplatte gegeben und dort mit einer zweiten Glasplatte abgedeckt, die durch Abstandshalter auf einen Abstand von 20 μm gehalten wurde. Dieser Probenkörper wurde 12 Stunden bei Raumtemperatur liegen gelassen und gehärtet.

Die Medien 2-8 wurden in analoger Art und Weise aus den in Tabelle 5 aufgeführten Beispielen hergestellt.

Die Medien 1-9 reflektieren nicht mehr die Erfindung, da sie keine fluorierten urethane als Weichmacher enthalten.

**Vergleichsmedium 1:**

6.12 g der wie oben beschrieben hergestellten Polyol-Komponente 2 wurden mit 3.75 g Phosphorothioyltris(oxybenzol-4,1-diylcarbamoyloxyethan-2,1-diyl)trisacrylat (Vergleichsbeispiel 1), 0.15 g CGI 909, 0.015 g Neu Methylenblau und 0.525 g N-Ethylpyrilidon bei 60 °C gemischt, so dass eine klare Lösung erhalten wurde. Anschließend wurde auf 30 °C abgekühlt, 4.418 g Baytec® WE 180 zugegeben und erneut gemischt. Schließlich wurden 0.030 g Fomrez® UL 28 zugegeben und erneut kurz gemischt. Die erhaltene, flüssige Masse wurde dann auf eine Glasplatte gegeben und dort mit einer zweiten Glasplatte abgedeckt, die durch Abstandshalter auf einen Abstand von 20 μm gehalten wurde. Dieser Probenkörper wurde bei Raumtemperatur liegen gelassen und über 16 Stunden gehärtet.

**Vergleichsmedium 2:**

8.89 g der wie oben beschrieben hergestellten Polyol-Komponente 1 wurden mit 3.75 g Propan-2,2-diylbis{(2,6-dibrombenzol-4,1-diyl)oxy-3-[(3-oxopent-4-en-1-yl)oxy]propan-1,2-diyl}bis[3,3,3-tris(4-chlorphenyl)propanoat] [1016639-27-2, vgl. WO 2008/125229 A1, $T_G$ + 47 °C], 0.15 g CGI 909, 0.015 g Neu Methylenblau und 0.525 g N-Ethylpyrilidon bei 60 °C gemischt, so dass eine klare Lösung erhalten wurde. Anschließend wurde auf 30 °C abgekühlt, 1.647 g Desmodur® N 3900 zugegeben und erneut gemischt. Schließlich wurden 0.006 g Fomrez UL 28 zugegeben und erneut kurz gemischt. Die erhaltene, flüssige Masse wurde dann auf eine Glasplatte gegeben und dort mit einer zweiten Glasplatte abgedeckt, die durch Abstandshalter auf einen Abstand von 20 μm gehalten wurde. Dieser Proben-körper wurde 12 Stunden bei Raumtemperatur liegen gelassen und gehärtet.

Die wie beschrieben hergestellten Medien wurden anschließend mittels einer Messanordnung gemäß Figur 1 wie folgt auf ihre holographischen Eigenschaften geprüft:

Dabei ergaben sich folgende Messwerte für $\Delta n_{sat}$ bei der Dosis E [mJ/cm$^2$]:

Tabelle 5: Holographische Bewertung ausgewählter Beispiele

| Medium | Acrylat aus Beispiel | $\Delta n_{sat}$ | Dosis [mJ/cm$^2$] |
|---|---|---|---|
| 1 | 9 | 0.0072 | 10 |
| 2 | 12 | 0.0083 | 18 |
| 3 | 17 | 0.0101 | 4 |
| 4 | 19 | 0.0086 | 9 |
| 5 | 20 | 0.0089 | 18 |
| 6 | 21 | 0.0079 | 18 |
| 7 | 24 | 0.0088 | 18 |
| 8 | 26 | 0.0078 | 9 |
| Vergleichsmedium 1 | Vergleichsbeispiel 1 | 0.0063 | 9 |
| Vergleichsmedium 2 | [1016639-27-2] | 0.0058 | 9 |

Die gefundenen Werte für den $\Delta n_{sat}$ bei der Dosis E zeigen, dass das im Vergleichsmedium verwendete Acrylat für die Verwendung in holographischen Medien weniger geeignet ist, wohingegen die Acrylate in den Medien 1 bis 8 für die Herstellung holographischer Medien aufgrund des höheren Wertes für $\Delta n_{sat}$ sehr gut geeignet sind. Zudem zeichnen sich die beschriebenen Acrylate relativ zum Vergleichsbeispiel durch eine niedrigere Glastemperatur $T_G$ aus, was die Löslichkeit der erfindungsgemässen (Meth)acrylate in einer resultierenden Photopolymer-Formulierung deutlich verbessert.

**Patentansprüche**

1. Photopolymer-Formulierung umfassend Matrixpolymere, Schreibmonomere und Photoinitiatoren, **dadurch gekennzeichnet, dass** die Schreibmonomere eine Verbindung ausgewählt aus der Liste 2-{[(Phenoxy)carbonyl]amino}ethylacrylat, 2- {[(2-Bromphenoxy)carbonyl]amino}ethylacrylat, 2- {[(3-Bromphenoxy)carbonyl]amino}ethyl-acrylat, 2-{[(4-Bromphenoxy)carbonyl]amino}ethylacrylat, 2- {[(3,4-Dichlorphenoxy)carbonyl]amino}ethylacrylat, 2-{[(2-Iodphenoxy)carbonyl]amino}ethylacrylat, 2-{[(3-Iodphenoxy)carbonyl]amino}ethylacrylat, 2-{[(4-Iodphenoxy)-carbonyl]amino}ethylacrylat, 2-{[(4-Brom-2-chlorphenoxy)carbonyl]amino}ethylacrylat, 2-{[(2-Brom-4-chlorphenoxy)carbonyl]amino}ethylacrylat, 2-{[(2,4-Dibromphenoxy)carbonyl]amino}ethylacrylat, 2-({[3-(Methylsulfanyl)phenoxy]carbonyl}amino)ethylacrylat, 2-({[2-(Methylsulfanyl)phenoxy]carbonyl}amino)ethylacrylat, 2-({[4-(Methylsulfanyl)phenoxy]carbonyl}amino)ethylacrylat, 2-{[(Biphenyl-4-yloxy)carbonyl]amino}ethylacrylat, 2-{[(Biphenyl-2-yloxy)carbonyl]amino}ethylacrylat, 2-{[(Biphenyl-3-yloxy)carbonyl]amino} ethylacrylat, 2-[(Phenylcarbamoyl)amino]ethylacrylat, 2- {[Methyl(phenyl)carbamoyl]amino}ethylacrylat, 2-{[(3-Bromphenyl)(methyl)carbamoyl]amino} ethylacrylat, 2-{[(3-Chlorphenyl)(methyl)carbamoyl]amino}ethylacrylat, 2-{[(3-Chlorphenyl)(ethyl)carbamoyl]-amino}ethylacrylat, 2-{[(2-Fluorphenyl)(methyl)carbamoyl]amino}ethylacrylat, 2-{[(4-Bromphenyl)(methyl)carbamoyl]amino}ethylacrylat, 2-{[(4-Chlorphenyl)(ethyl)carbamoyl]amino}ethylacrylat, 2-{[(4-Chlorphenyl)(methyl)carbamoyl]amino}ethylacrylat, 2-({Methyl[3-(methylsulfanyl)phenyl]carbamoyl}amino)ethylacrylat, 2-{[(Phenoxy)carbonyl]amino}ethylmethacrylat, 2-{[(3-Bromphenoxy)carbonyl]amino}ethylmethacrylat, 2-{[(3,4-Dichlorophenoxy)carbonyl]amino}ethylacrylat, 2-{[(3-Iodophenoxy)carbonyl]amino}ethylmethacrylat, 2-{[(3-Chloro-4-bromophenoxy)carbonyl]amino}ethylmethacrylat, 2-{[(2-Bromo-4-chlorophenoxy)carbonyl]amino}ethylmethacrylat, 2-{[(2,4-Dibromo-phenoxy)carbonyl]amino} ethylacrylat, 2-{[(3-Methylthiophenoxy)carbonyl]amino}ethylmethacrylat, 2-{[(4-Phenylphenoxy)carbonyl]amino}ethylmethacrylat, 2-{[(2-Phenylphenoxy)carbonyl]amino}ethylmethacrylat, 2- {[(3-Phenylphenoxy)carbonyl]amino}ethylmethacrylat, 2-{[Methyl(phenyl)carbamoyl]amino}2-methylprop-2-enoat, 2-{[(3-Chlorophenyl)(methyl)carbamoyl]amino}ethyl-2-methylprop-2-enoat, 2- {[(2-Fluorophenyl)(methyl)carbamoyl]amino}ethyl-2-methylprop-2-enoat, 2-{[(4-Bromophenyl)(methyl)carbamoyl]amino}ethyl-2-methylprop-2-enoat, 2- {[(4-Chlorophenyl)(ethyl)carbamoyl]amino}ethyl-2-methylprop-2-enoat, 2- {[(4-Chlorophenyl)(methyl)carbamoyl]amino}ethyl-2-methylprop-2-enoat enthalten, und die Photopolymer-Formulierung zusätzlich Urethane als Weichmacher enthält, wobei die Urethane mit wenigstens einem Fluoratom substituiert sind.

**2.** Photopolymer-Formulierung nach Anspruch 1, **dadurch gekennzeichnet, dass** die Matrixpolymere Polyurethane sind.

**3.** Photopolymer-Formulierung nach einem der Ansprüche 1 oder 2, **dadurch gekennzeichnet, dass** die Photoinitiatoren einen anionischen, kationischen oder neutralen Farbstoff und einen Coinitiator umfassen.

**4.** Verwendung einer Photopolymer-Formulierung nach einem der Ansprüche 1 bis 3 zur Herstellung holographischer Medien, insbesondere zur Herstellung von In-Line Hologrammen, Off-Axis Hologrammen, Full-Aperture Transfer Hologrammen, Weißlicht-Transmissionshologrammen, Denisyukhologrammen, Off-Axis Reflektionshologrammen, Edge-Lit Hologrammen sowie holographischen Stereogrammen.

## Claims

**1.** Photopolymer formulation comprising matrix polymers, writing monomers and photoinitiators, **characterized in that** the writing monomers contain a compound selected from the list 2-{[(phenoxy)carbonyl]amino}ethyl acrylate, 2-{[(2-bromophenoxy)carbonyl]amino}ethyl acrylate, 2-{[(3-bromophenoxy)carbonyl]amino}ethyl acrylate, 2-{[(4-bromophenoxy)carbonyl]amino}ethyl acrylate, 2-{[(3,4-dichlorophenoxy)carbonyl]amino}ethyl acrylate, 2-{[(2-iodophenoxy)carbonyl]amino}ethyl acrylate, 2-{[(3-iodophenoxy)carbonyl]amino}ethyl acrylate, 2-{[(4-iodophenoxy)carbonyl]amino}ethyl acrylate, 2-{[(4-bromo-2-chlorophenoxy)carbonyl]-amino}ethyl acrylate, 2-{[(2-bromo-4-chlorophenoxy)carbonyl]amino}ethyl acrylate, 2-{[(2,4-dibromophenoxy)carbonyl]amino}ethyl acrylate, 2-({[3-(methylsulphanyl)phenoxy]carbonyl}amino)ethyl acrylate, 2-({[2-(methylsulphanyl)phenoxy]carbonyl}amino)ethyl acrylate, 2-({[4-(methylsulphanyl)-phenoxy]carbonyl}amino)ethyl acrylate, 2-{[(biphenyl-4-yloxy)carbonyl]amino}ethyl acrylate, 2-{[(biphenyl-2-yloxy)carbonyl]-amino}ethyl acrylate, 2-{[(biphenyl-3-yloxy)carbonyl]amino}ethyl acrylate, 2-[(phenylcarbamoyl)amino]ethyl acrylate, 2-{[methyl(phenyl)carbamoyl]amino}ethyl acrylate, 2-{[(3-bromophenyl)(methyl)carbamoyl]amino}ethyl acrylate, 2-{[(3-chlorophenyl)(methyl)carbamoyl]amino}ethyl acrylate, 2-{[(3-chlorophenyl)(ethyl)carbamoyl]amino}ethyl acrylate, 2-{[(2-fluorophenyl)(methyl)carbamoyl]amino}ethyl acrylate, 2-{[(4-bromophenyl)(methyl)carbamoyl]amino}ethyl acrylate, 2-{[(4-chlorophenyl)(ethyl)carbamoyl]-amino}ethyl acrylate, 2-{[(4-chlorophenyl)(methyl)carbamoyl]amino}ethyl acrylate, 2-({methyl[3-(methylsulphanyl)phenyl]carbamoyl}amino)ethyl acrylate, 2-{[(phenoxy)carbonyl]amino}ethyl methacrylate, 2-{[(3-bromophenoxy)carbonyl]amino}ethyl methacrylate, 2-{[(3,4-dichlorophenoxy)carbonyl]amino}ethyl acrylate, 2-{[(3-iodophenoxy)carbonyl]amino}ethyl methacrylate, 2-{[(3-chloro-4-bromophenoxy)carbonyl]amino}ethyl methacrylate, 2-{[(2-bromo-4-chlorophenoxy)carbonyl]amino}ethyl methacrylate, 2-{[(2,4-dibromophenoxy)-carbonyl]amino}ethyl acrylate, 2-{[(3-methylthiophenoxy)carbonyl]amino}ethyl methacrylate, 2-{[(4-phenylphenoxy)carbonyl]amino}ethyl methacrylate, 2-{[(2-phenylphenoxy)carbonyl]amino}ethyl methacrylate, 2-{[(3-phenylphenoxy)carbonyl]amino}ethyl methacrylate, 2-{[methyl(phenyl)carbamoyl]amino} 2-methylprop-2-enoate, 2-{[(3-chlorophenyl)(methyl)carbamoyl]amino}ethyl 2-methylprop-2-enoate, 2-{[(2-fluorophenyl)-(methyl)carbamoyl]amino}ethyl 2-methylprop-2-enoate, 2-{[(4-bromophenyl)(methyl)carbamoyl]amino}ethyl 2-methylprop-2-enoate, 2-{[(4-chlorophenyl)(ethyl)-carbamoyl]amino}ethyl 2-methylprop-2-enoate, 2-{[(4-chlorophenyl(methyl)carbamoyl]amino}ethyl 2-methylprop-2-enoate, and the photopolymer formulation additionally contains urethanes as plasticizers, wherein the urethanes are substituted with at least a fluorine atom.

**2.** Photopolymer formulation according to Claim 1, **characterized in that** the matrix polymers are polyurethanes.

**3.** Photopolymer formulation according to either of Claims 1 and 2, **characterized in that** the photoinitiators comprise an anionic, cationic or neutral dye and a co-initiator.

**4.** Use of a photopolymer formulation according to any of Claims 1 to 3 in the manufacture of holographic media, in particular for production of In-Line holograms, Off-Axis holograms, Full-Aperture Transfer holograms, white light transmission holograms, Denisyuk holograms, Off-Axis reflection holograms, Edge-Lit holograms and also holographic stereograms.

**EP 2 354 845 B1**

**Revendications**

1. Formulation de photopolymère comprenant des polymères de matrice, des monomères d'enregistrement et des photo-initiateurs, **caractérisée en ce que** les monomères d'enregistrement contiennent un composé, choisi parmi la liste acrylate de 2-{[(phénoxy)carbonyl]amino}éthyle, acrylate de 2-{[(2-bromophénoxy)carbonyl]amino}éthyle, acrylate de 2-{[(3-bromophénoxy)carbonyl]amino}éthyle, acrylate de 2-{[(4-bromophénoxy)carbonyl]amino}éthyle, acrylate de 2-{[(3,4-dichlorophénoxy)carbonyl]amino}éthyle, acrylate de 2-{[(2-iodophénoxy)carbonyl]amino}éthyle, acrylate de 2-{[(3-iodophénoxy)carbonyl]amino}éthyle, acrylate de 2-{[(4-iodophénoxy)-carbonyl]amino}éthyle, acrylate de 2-{[(4-bromo-2-chlorophénoxy)carbonyl]amino}éthyle, acrylate de 2-{[(2-bromo-4-chlorophénoxy)carbonyl]amino}éthyle, acrylate de 2-{[(2,4-dibromophénoxy)carbonyl]amino}éthyle, acrylate de 2-({[3-(méthylsulfanyl)phénoxy]carbonyl}amino)éthyle, acrylate de 2-({[2-(méthylsulfanyl)phénoxy]carbonyl}amino)éthyle, acrylate de 2-({[4-(méthylsulfanyl)phénoxy]carbonyl}amino)éthyle, acrylate de 2-{[(biphényl-4-yloxy)carbonyl]amino}éthyle, acrylate de 2-{[(biphényl-2-yloxy)carbonyl]amino}éthyle, acrylate de 2-{[(biphényl-3-yloxy)carbonyl]amino}éthyle, acrylate de 2-[(phénylcarbamoyl)amino]éthyle, acrylate de 2-{[méthyl(phényl)carbamoyl]amino}éthyle, acrylate de 2-{[(3-bromophényl)(méthyl)carbamoyl]amino}éthyle, acrylate de 2-{[(3-chlorophényl)(méthyl)carbamoyl]amino}éthyle, acrylate de 2-{[(3-chlorophényl)(éthyl)carbamoyl]-amino}éthyle, acrylate de 2-{[(2-fluorophényl)(méthyl)carbamoyl]amino}éthyle, acrylate de 2-{[(4-bromophényl)(méthyl)carbamoyl]amino}éthyle, acrylate de 2-{[(4-chlorophényl)(éthyl)carbamoyl]amino}éthyle, acrylate de 2-{[(4-chlorophényl)(méthyl)carbamoyl]amino}éthyle, acrylate de 2-({méthyl[3-(méthylsulfanyl)phényl]carbamoyl}amino)éthyle, méthacrylate de 2-{[(phénoxy)carbonyl]amino}éthyle, méthacrylate de 2-{[(3-bromophénoxy)carbonyl]amino}éthyle, méthacrylate de 2-{[(3,4-dichlorophénoxy)carbonyl]amino}éthyle acrylate, méthacrylate de 2-{[(3-iodophénoxy)carbonyl]amino}éthyle, méthacrylate de 2-{[(3-chloro-4-bromophénoxy)carbonyl]amino}éthyle, méthacrylate de 2-{[(2-bromo-4-chlorophénoxy)carbonyl]amino}éthyle, méthacrylate de 2-{[(2,4-dibromophénoxy)carbonyl]amino}éthyle acrylate, méthacrylate de 2-{[(3-méthylthiophénoxy)carbonyl]amino}éthyle, méthacrylate de 2-{[(4-phénylphénoxy)carbonyl]amino}éthyle, méthacrylate de 2-{[(2-phénylphénoxy)carbonyl]amino}éthyle, méthacrylate de 2-{[(3-phénylphénoxy)carbonyl]amino}éthyle, prop-2-énoate de 2-{[méthyl(phényl)carbamoyl]amino}2-méthyle, prop-2-énoate de 2-{[(3-chlorophényl)(méthyl)carbamoyl]amino}éthyl-2-méthyle, prop-2-énoate de 2-{[(2-fluorophényl)(méthyl)carbamoyl]amino}éthyl-2-méthyle, prop-2-énoate de 2-{[(4-bromophényl)(méthyl)carbamoyl]amino}éthyl-2-méthyle, prop-2-énoate de 2-{[(4-chlorophényl)(éthyl)carbamoyl]amino}éthyl-2-méthyle, prop-2-énoate de 2-{[(4-chlorophényl)(méthyl)carbamoyl]amino}éthyl-2-méthyle, et la formulation de photopolymères contient en outre des uréthanes comme plastifiants, les uréthanes étant substitués par au moins un atome de fluor.

2. Formulation de photopolymère selon la revendication 1, **caractérisée en ce que** les polymères de matrice sont des polyuréthanes.

3. Formulation de photopolymère selon l'une quelconque des revendications 1 ou 2, **caractérisée en ce que** les photo-initiateurs comprennent un colorant anionique, cationique ou neutre et un co-initiateur.

4. Utilisation d'une formulation de photopolymère selon l'une quelconque des revendications 1 à 3 pour la production d'agents holographiques, en particulier pour la production d'hologrammes en ligne, d'hologrammes hors axe, d'hologrammes de transfert à pleine ouverture, d'hologrammes de transmission en lumière blanche, d'hologrammes de Denisyuk, d'hologrammes de réflexion hors axe, d'hologrammes à éclairage périphérique ainsi que de stéréogrammes holographiques.

**Fig. 1**

$\Delta n = 0.0108$
$d' = 22~\mu m$
$E = 9.1~mJ/cm^2$

**Fig. 2**

## IN DER BESCHREIBUNG AUFGEFÜHRTE DOKUMENTE

**In der Beschreibung aufgeführte Patentdokumente**

- WO 2008125229 A1
- JP 2007101743 A
- EP 0223587 A

**In der Beschreibung aufgeführte Nicht-Patentliteratur**

- **P. HARIHARAN.** Optical Holography. Cambridge University Press, 1996
- **CUNNINGHAM et al.** *RadTech'98 North America UV/EB Conference Proceedings,* 19. April 1998
- **KUTAL et al.** *Macromolecules,* 1991, vol. 24, 6872
- **YAMAGUCHI et al.** *Macromolecules,* 2000, vol. 33, 1152
- **NECKERS et al.** *Macromolecules,* 2000, vol. 33, 7761
- **DEKTAR et al.** *J. Org. Chem.,* 1990, vol. 55, 639
- *J. Org. Chem.,* 1991, vol. 56, 1838
- **CRIVELLO et al.** *Macromolecules,* 2000, vol. 33, 825
- **GU et al.** *Am. Chem. Soc. Polymer Preprints,* 2000, vol. 41 (2), 1266
- **HUA et al.** *Macromolecules,* 2001, vol. 34, 2488-2494
- Chemistry & Technology of UV & EB Formulations For Coatings. Inks & Paints. SITA Technology, 1991, vol. 3, 61-328
- **NG, WILLIE W. ; HONG, CHI-SHAIN ; YARIV, AMNON.** Holographic interference lithography for integrated optics. *IEEE Transactions on Electron Devices,* 1978, vol. ED-25 (10), ISSN 0018-9383, 1193-1200